# EUROPÄISCHE PATENTSCHRIFT

(11) **EP 0 802 779 B1**
(45) Veröffentlichungstag und Bekanntmachung des Hinweises auf die Patenterteilung: **11.08.1999**
(21) Anmeldenummer: 95943203.0
(22) Anmeldetag: 22.12.1995
(51) Int. Cl.: A61J 3/10, A61K 9/20, B30B 11/16

(54) **VERFAHREN UND VORRICHTUNG ZUR HERSTELLUNG VON LINSENFÖRMIGEN TABLETTEN DURCH SCHMELZKALANDRIERUNG**
METHOD AND APPARATUS FOR PRODUCING LENTICULAR TABLETS BY MELT CALENDERING
PROCEDE ET APPAREIL DE FABRICATION DE COMPRIMES LENTICULAIRES PAR CALANDRAGE D'UNE MATIERE FONDUE

(30) Priorität: 23.12.1994 DE 4446467
(43) Veröffentlichungstag der Anmeldung: 29.10.1997
(73) Patentinhaber: BASF AKTIENGESELLSCHAFT, 67056 Ludwigshafen (DE)
(72) Erfinder: ROSENBERG, Joerg, D-67158 Ellerstadt (DE); MAIER, Werner, D-67105 Schifferstadt (DE); BREITENBACH, Jörg, D-68199 Mannheim (DE)
(74) Vertreter: Kinzebach, Werner, Dr.
(86) Internationale Anmeldenummer: EP9505119
(87) Internationale Veröffentlichungsnummer: WO9619964

(56) Entgegenhaltungen:
- DE-A- 1 766 546
- DE-A- 3 830 355
- US-A- 4 880 585
- AUFBEREITUNGS-TECHNIK, Bd. 11, Nr. 3, März 1970, Seiten 128-138, XP002000518 PIETSCH: "die bedeutung der walzenkonstruktion u.s.w."

## Beschreibung

Die vorliegende Erfindung betrifft ein Verfahren zur Herstellung von linsenförmigen Tabletten durch Formen einer wirkstoffhaltigen Schmelze in einem Kalander mit gegenläufig rotierenden Formwalzen, die auf ihrer Oberfläche Vertiefungen zur Aufnahme und Formung der Schmelze aufweisen (Schmelzkalandrierung) und eine Kalander Formwalze zur Durchführung des Verfahrens.

Die Herstellung von Tabletten durch Kalandrierung einer wirkstoffhaltigen Schmelze ist aus der DE-A- 1 766 546 und der US-A-4,880,585 bekannt. Grundlage dieses Verfahren ist die Einbettung eines Wirkstoffes in eine Schmelze aus einem Träger, z.B. Fettsubstanzen oder wasserlösliche, thermoplastische Polymere. Die Schmelze wird dadurch erzeugt, daß die Mischung aus Wirkstoff, Polymer und gegebenenfalls weiteren Hilfsstoffen beispielsweise in einem Extruder aufgeschmolzen und als Schmelze in einem nachgeschalteten Formkalander zu Tabletten geformt wird, die durch Abkühlen aushärten. Der Formkalander umfaßt ein sich gegenläufig drehendes Formwalzenpaar, wobei die Formwalzen auf ihrer Oberfläche Gravuren (Vertiefungen) aufweisen, die der Form einer Hälfte der gewünschten Tablette entsprechen. Die Tablettenformung erfolgt im Berührungsbereich der beiden Walzen durch Kombination der Tablettenmasse einer Vertiefung auf der einen Walze mit derjenigen der gegenüberliegenden Vertiefung auf der anderen Walze. Sowohl in der DE-A-1 766 546 als auch in der US-A-4,880 585 ist die Herstellung stäbchenförmiger Tabletten (Oblong-Tabletten) beschrieben.

Bei der Herstellung von Tabletten nach dem Schmelzkalandrierverfahren entsteht in der Regel als Folge des Preßvorgangs eine umlaufende Preßnaht, die aus Schmelzeresten besteht. Diese Preßnaht muß nach dem Erkalten der Tabletten durch Entgratungstechniken entfernt werden. Dies kann bei harten, spröden Tablettenformulierungen und dünnen Preßnähten auf einfache Weise beispielsweise dadurch erfolgen, daß man die Tabletten in rotierende Kessel gibt, in denen durch gegenseitiges Abschleifen der Tabletten untereinander eine Entgratung erfolgt. Bei dicken Preßnahten oder bei plastisch verformbaren Formulierungen ist eine einfache Entgratung nicht möglich. Beispielsweise ist eine Entgratung der in der US-A-4,880,585 beschriebenen Oblong-Tabletten nach dem erwähnten einfachen Verfahren in rotierenden Kesseln nicht zufriedenstellend möglich. Um in solchen Fällen eine ausreichende Entgratung sicherzustellen, muß auf andere Entgratungstechniken ausgewichen werden, welche die Herstellungskosten erhöhen.

Auf Grund von Ungenauigkeiten bei der Herstellung der Formwalzen oder ungleichmäßiger Rotation der Formwalzen liegen häufig die zur Formung einer Tablette erforderlichen Vertiefungen einander nicht exakt gegenüber. Das hat zur Folge, daß die beiden Tablettenhälften gegeneinander versetzt vorliegen.

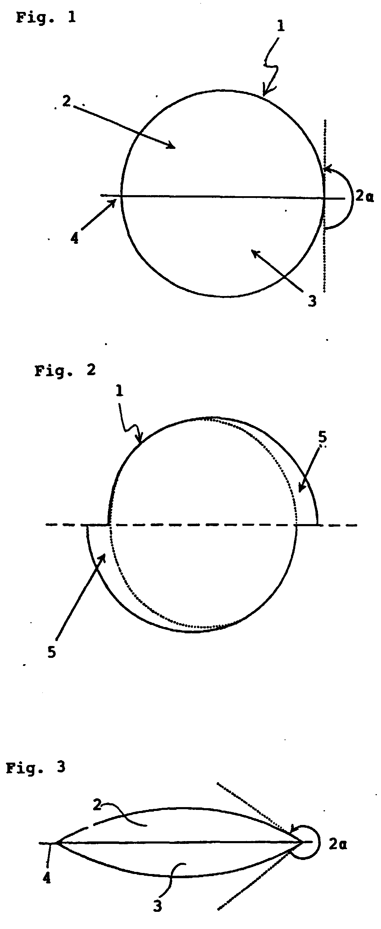

Ein derartiger Versatz zwischen Tablettenober- und Tablettenunterhälfte bei den Oblong-Tabletten gemäß US-A-4,880,585 ist in Figur 2 gezeigt. Um brauchbare Tabletten zu erhalten, müssen die überstehenden Tablettenreste abgeschliffen werden. Aus Figur 2 ist ersichtlich, daß bei den Oblong-Tabletten des Standes der Technik große Mengen an Tablettenmaterial abgeschliffen werden müssen, was äußerst zeitaufwendig und in der Regel mit einem Materialverlust verbunden ist. In der DE-A-38 30 355 ist ein Weg zur Vermeidung der oben genannten Schwierigkeiten insofern vorgeschlagen, als die Tabletten nicht durch Kalandrieren sondern nach dem Extrudieren in einer konventionellen Tablettiermaschine bei 25°C geformt werden.

Der vorliegenden Erfindung liegt die Aufgabe zugrunde, ein Verfahren zur Herstellung von Tabletten und eine Kalanderformwalze zur Durchführung des Verfahrens zur Verfügung zu stellen, bei der die Entgratung der Tabletten bzw. die Entfernung der durch einen Versatz zwischen den Tablettenhälften überstehenden Teile auf einfache Weise möglich ist.

Die Aufgabe wird durch ein Verfahren nach Anspruch 1 und eine Kalanderformwalze nach Anspruch 7 gelöst.

### Beschreibung der Zeichnung:

Figur 1 ist eine Ansicht einer Oblong-Tablette gemäß US-A-4,880,585 mit einer seitlich abstehenden Preßnaht.
Figur 2 ist ein Querschnitt durch eine entsprechende Oblong-Tablette, wobei die Tablettenober- und -unterhälfte gegeneinander versetzt sind.
Figur 3 ist die Seitenansicht einer erfindungsgemäß erhältlichen Tablette mit Preßnaht.
Figur 4 ist ebenfalls eine Seitenansicht durch eine erfindungsgemäß erhältliche Tablette, wobei die Tablettenober- und -unterhälfte gegeneinander versetzt angeordnet sind.

Die sich entlang einer Mantellinie berührenden Formwalzen des Kalanders enthalten, wie erwähnt, Vertiefungen in Form eines Ellipsoidsegments, welche jeweils einer Tablettenhälfte entsprechen.

Mit dem erfindungsmäßen Verfahren erhält man aufgrund der ellipsoidsegmentartigen Vertiefungen in den Formwalzen flache linsenförmige Tabletten. Vorzugsweise liegen diese Vertiefungen in Form eines Kugelsegments vor, so daß die erhaltenen Tabletten rund sind.

Die Preßnaht liegt bei den erfindungsgemäß erhaltenen Tabletten als flanschartiger Rand vor, der sich in der Mittelebene der Tabletten erstreckt. Der Winkel α zwischen der Ebene der Preßnaht und dem Tablettenkörper (Tangentialfläche an die Preßnaht) ist größer als 135°. In entsprechender Weise ist der Winkel 2α zwischen oberer und unterer Tangentialfläche an der Preßnaht größer als 270°, siehe Figur 3. Entsprechend ist bei den Vertiefungen der Winkel zwischen der Tangentialfläche der Vertiefung und der Tangentialfläche der Walze im Schnittpunkt der Vertiefung mit der Walzenoberfläche kleiner als 45°.

Im Gegensatz dazu beträgt bei der Oblong-Tablette des Standes der Technik der Winkel α zwischen der Ebene der Preßnaht und dem Tablettenkörper etwa 90° bzw. der Winkel zwischen der oberen und unteren Tangentialfläche an die Preßnaht etwa 180°, siehe Figur 1.

Dies hat erhebliche Konsequenzen für die nachfolgenden Entgratungsschritte. Während im Falle der Oblong-Tabletten lange Entgratungszeiten benötigt werden, reduziert sich dieser Aufwand bei den erfindungsgemäß erhältlichen Tabletten erheblich, da wegen des größeren Winkels sowohl die Häufigkeit (bessere Zugänglichkeit) als auch die Intensität des gegenseitigen Kontaktes zwischen den Tabletten beim Entgraten erheblich steigen. Die relativ spitzen, scharfen Außenkanten der noch nicht entgrateten, erfindungsgemäß erhältlichen Tabletten werden beim Entgraten zusätzlich abgerundet, so daß sich letztendlich Tabletten herstellen lassen, die in Form und Aussehen den konventionell, d.h. durch Verpressen von Granulaten, hergestellten Tabletten gleich sind.

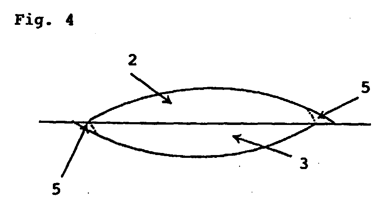

Ein weiterer Vorteil der erfindungsgemäß erhältlichen Tabletten besteht darin, daß das bei einem Versatz zwischen oberer und unterer Tablettenhälfte abzuschleifende Tablettenmaterial sehr gering ist, wie der Figur 4 zu entnehmen ist. Dies hat zur Folge, daß einerseits der Zeitaufwand für das Abschleifen des Tablettenmaterials erheblich geringer ist und daß andererseits die Anforderungen an die Präzision und Rotation der eingesetzten Formkalanderwalzen verringert werden können, wodurch die Maschinenkosten sinken.

Das Problem des Versatzes wird vollständig vermieden, wenn eine Formwalze mit ellipsoidartigen Vertiefungen mit einer Glattwalze kombiniert wird. Man erhält auf diese Weise Tabletten in Form eines Ellipsoidsegments bzw. Kugelsegments, das einer Tablettenhälfte z.B. gemäß Figur 3 entspricht. Diese Tablette läßt sich ebenso leicht entgraten wie beispielsweise die Tablette gemäß Figur 3.

Gewünschtenfalls können auch teilbare Tabletten hergestellt werden. Zu diesem Zweck kann man am Boden der Vertiefungen eine kleine oft im Mikrometerbereich liegende Rippe vorsehen, die zur Ausbildung der Bruchrille in den fertiggestellten Tabletten führt. Vorzugsweise aber verwendet man wenigstens eine Formwalze, bei der die Vertiefungen durch mindestens einen Steg unterteilt sind, der sich im wesentlichen bis zur Mantelfläche der Formwalze erstreckt und die Ausbildung der Bruchrille bewirkt.

Die Herstellung der Tabletten erfolgt ausgehend von einer Mischung, die einen oder mehrere pharmazeutische Wirkstoffe sowie einen oder mehrere übliche Hilfsstoffe enthält und die durch Schmelzen oder Erweichen mindestens einer Komponente teigig bis zähflüssig und daher extrudierbar wird.

Das sind insbesondere Mischungen die pharmakologisch akzeptable Polymere enthalten (wobei die Glastemperatur der Mischung unter der Zersetzungstemperatur aller Mischungskomponenten liegt), beispielsweise Polyvinylpyrrolidon (PVP), Copolymerisate von N-Vinylpyrrolidon (NVP) und Vinylacetat, Copolymerisate von Vinylacetat und Crotonsäure, teilverseiftes Polyvinylacetat, Polyvinylalkohol, Ethylen/Vinylacetat-Copolymerisate, Polyhydroxyethylmethacrylat, Copolymerisate von Methylmethacrylat und Acrylsäure, Celluloseester, Celluloseether, Polyethylenglykol oder Polyethylen, bevorzugt NVP-Copolymerisate mit Vinylacetat, Hydroxypropylcellulose und Polyethylenglycole/Polyethylenoxide. Die K-Werte (nach H. Fikentscher, Cellulose-Chemie 13 (1932), Seiten 58 bis 64 und 71 und 74) der Polymeren liegen im Bereich von 10 bis 100, vorzugsweise 12 bis 70, insbesondere 12 bis 35, für PVP vorzugsweise bei 12 bis 35, insbesondere bei 12 bis 17.

Das polymere Bindemittel muß in der Gesamtmischung aller Komponenten im Bereich von 50 bis 180, vorzugsweise 60 bis 130°C erweichen oder schmelzen, so daß die Masse extrudierbar ist. Die Glasübergangstemperatur der Mischung muß also auf jeden Fall unter 180, vorzugsweise unter 130°C liegen. Erforderlichenfalls wird sie durch übliche pharmakologisch akzeptable weichmachende Hilfsstoffe wie langkettige Alkohole, Ethylenglykol, Propylenglykol, Trimethylolpropan, Triethylenglykol, Butandiole, Pentanole, Hexanole, Polyethylenglykole, Silicone, aromatische Carbonsäureester (z.B. Dialkylphthalate, Trimellithsäureester, Benzoesäureester, Terephthalsäureester) oder aliphatische Dicarbonsäureester (z.B. Dialkyladipate, Sebacinsäureester, Azelainsäureester, Zitronen- und Weinsäureester) oder Fettsäureester herabgesetzt.

Übliche galenische Hilfsstoffe, deren Gesamtmenge bis zu 100 Gew.-% bezogen auf das Polymerisat, betragen kann, sind z.B. Streckmittel wie Silikate oder Kieselerde, Stearinsäure oder deren Salze, z.B. das Magnesium- oder Kalziumsalz, Methylcellulose, Natrium-Carboxymethylcellulose, Talkum, Saccharose, Lactose, Getreide- oder Maisstärke, Kartoffelmehl, Polyvinylalkohol, ferner Netz-, Konservierungs-, Spreng-, Adsorptionsmittel, Farbstoffe, Geschmacksstoffe (vgl. z.B. H. Sucker et al. Pharmazeutische Technologie, Thieme-Verlag, Stuttgart 19/8). Sie müssen bei den hier zur Anwendung kommenden Temperaturen thermisch stabil sein.

Unter pharmazeutischen Wirkstoffen im Sinne der Erfindung sind alle Stoffe mit einer pharmazeutischen Wirkung und möglichst geringen Nebenwirkungen zu verstehen, sofern sie sich unter den Verarbeitungsbedingungen nicht zersetzen. Die Wirkstoffmenge pro Dosiseinheit und die Konzentration können je nach Wirksamkeit und Freisetzungsgeschwindigkeit in weiten Grenzen variieren. Die einzige Bedingung ist, daß sie zur Erzielung der gewünschten Wirkung ausreichen. So kann die Wirkstoffkonzentration im Bereich von 0,1 bis 95, vorzugsweise von 20 bis 80, insbesondere 30 bis 70 Gew.-% liegen. Auch Wirkstoff-Kombinationen können eingesetzt werden. Wirkstoffe im Sinne der Erfindung sind auch Vitamine sowie Pflanzenbehandlungsmittel und Insektizide.

Das erfindungsgemäße Verfahren ist beispielsweise zur Verarbeitung folgender Wirkstoffe geeignet:

Acebutolol, Acetylcystein, Acetylsalicylsäure, Acyclovir, Albrazolam, Alfacalcidol, Allantoin, Allopurinol, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkonium Hydroxid, Benzocain, Benzoesäure, Betamethason, Bezafibrat, Biotin, Biperiden, Bisoprolol, Prazosin, Bromazepam, Bromhexin, Bromocriptin, Budesonid, Bufexamac, Buflomedil, Buspiron, Coffein, Campher, Captopril, Carbamazepin, Carbidopa, Carboplatin, Carotinoide wie beispielsweise β-Carotin oder Canthaxanthin, Cefachlor, Cefalexin, Cefatroxil, Cefazolin, Cefixim, Cefotaxim, Ceftazidim, Ceftriaxon, Cefuroxim, Celedilin, Chloramphenicol, Chlorhexidin, Chlorpheniramin, Chlortalidon, Cholin, Cyclosporin, Cilastatin, Cimetidin, Ciprofloxacin, Cisapride, Cisplatin, Clarithromycin, Clävulansäure, Clomibramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Dihydrocodein, Dihydroergotamin, Diltiazem, Diphenhydramin, Dipyridamol, Dipyron, Disopyramid, Domperidon, Dopamin, Enalapril, Ephedrin, Epinephrin, Ergocalciferol, Ergotamin, Erythromycin, Estradiol, Ethinylestradiol, Etoposide, Eucalyptus Globulus, Famotidin, Felodipin, Fenofibrat, Fenoterol, Fentanyl, Flavin-Mononucleotid, Fluconazol, Flunarizin, Fluorouracil, Fluoxetin, Flurbiprofen, Furosemid, Gemfibrozil, Gentamicin, Ginkgo Biloba, Glibenclamid, Glipizid, Clozapin, Glycyrrhiza glabra, Guaifenesin, Haloperidol, Heparin, Hyaluronsäure, Hydrochlorothiazid, Hydrocodon, Hydrocortison, Hydromorphon, Ipratropium Hydroxid, Ibuprofen, Imipenem, Indomethacin, Iohexol, Iopamidol, Isosorbid-Dinitrat, Isosorbid-Mononitrat, Isotretinoin, Ketotifen, Ketoconazol, Ketoprofen, Ketorolac, Labatalon, Lactulose, Lecithin, Levocarnitin, Levodopa, Levoglutamide, Levonorgestrel, Levothyroxin, Lidocain, Lipase, Liponsäure, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. -kombinationen und Mineralsalze, N-Methylephedrin, Naftidrofuryl, Naproxen, Neomycin, Nicardipin, Nicergolin, Nicotinamid, Nicotin, Nicotinsäure, Nifedipin, Nimodipin, Nitrendipin, Nizatidin, Norethisteron, Norfloxacin, Norgestrel, Nortriptylin, Nystatin, Ofloxacin, Omeprazol, Ondanseron, Pancreatin, Panthenol, Pantothensäure, Paracetamol, Penicillin G, Penicillin V, Phenobarbital, Phenoxifyllin, Phenylephrin, Phenylpropanolamin, Phenytoin, Piroxicam, Polymyxin B, Povidone-Iod, Pravastatin, Prednisolon, Bromocriptin, Propafenon, Propranolol, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Simvastatin, Somatropin, Sotalol, Spironolacton, Sucralfat, Sulbactam, Sulfamethoxazol, Sulpiridid, Tamoxifen, Tegafur, Teprenon, Terazosin, Terbutalin, Terfenadin, Theophyllin, Thiamin, Tiolopidin, Timolol, Tranexamsäure, Tretinoin, Triamcinolon-Acetonid, Triamteren, Trimethoprim, Troxerutin, Uracil, Valproinsäure, Vancomycin, Verapamil, Vitamin B₁, B₂, B₄, B₆, B₁₂, D₃, E, K, Volinsäure, Zidovudin.

In Einzelfällen kommt es zur Bildung sogenannter "fester Lösungen". Der Begriff "feste Lösungen" ist dem Fachmann geläufig, beispielsweise aus der eingangs zitierten Literatur. In festen Lösungen von pharmazeutischen Wirkstoffen in Polymeren liegt der Wirkstoff molekulardispers im Polymer vor.

Die pharmazeutische Mischung wird dann in üblicher Weise aufgeschmolzen, vorzugsweise in einem Extruder, und dem Formkalander zugeführt, wie das beispielsweise in der US-A-4,880,585 beschrieben ist. Falls erforderlich werden die Tabletten nach der Kalandrierung gekühlt, z.B. in einem Luft- oder Kühlbad.

Bei klebrigen oder hochviskosen Materialien, die sich nur schwer oder gar nicht von der Form lösen, ist die Anwendung eines Formtrennmittels, beispielsweise ein Silikonöl oder ein Silikonlack, oder auch Mono-, Di- und Triglyceride und Lecithine, zweckmäßig.

Nachfolgend wird anhand der Figur und der Beispiele die Erfindung erläutert, ohne sie zu begrenzen.

Die Figur 1 zeigt eine Oblong-Tablette 1, die nach dem in der US-A-4,880,585 beschriebenen Verfahren erhalten worden ist: Sie besitzt eine umlaufende Preßnaht 4, welche die Tablette in eine Tablettenoberhälfte 2 und eine Tablettenunterhälfte 3 teilt. Der Winkel α zwischen der Ebene der Preßnaht und dem Tablettenkörper (Tangentialfläche an die Preßnaht) beträgt etwa 180°.

Die Figur 2 zeigt einen Querschnitt durch eine entsprechende Oblong-Tablette 1, bei der die obere Tablettenhälfte gegen die untere Tablettenhälfte versetzt ist. Es ist ersichtlich, daß der für die Erzielung einer Tablette mit gleichmäßiger Oberfläche abzuschleifende Tablettenrest 5 beträchtlich ist.

Die Figur 3 ist eine Seitenansicht einer Tablette, die nach dem erfindungsgemäßen Verfahren unter Verwendung von zwei Formwalzen mit kugelsegmentartigen Vertiefungen erhalten worden ist. Die Preßnaht 4 teilt die Tablette in eine Tablettenoberhälfte 2 und eine Tablettenunterhälfte 3. Der Winkel 2α zwischen oberer und unterer Tangentialfläche an der Preßnaht beträgt ca. 290°.

Die Figur 4 ist eine Seitenansicht einer Tablette, die im wesentlichen derjenigen der Figur 3 entspricht, wobei jedoch die obere Tablettenhälfte 2 gegenüber der unteren Tablettenhälfte 3 versetzt ist. Der abzuschleifende Tablettenrest ist dabei relativ gering.

### Beispiel 1

Tabletten dieser Art wurden ausgehend von einer Mischung hergestellt, die aus 60,0 Gew.-% Kollidon VA-64 (BASF) (Polyvinylpyrrolidon-Copolymer mit Vinylacetat (60:40)) besteht. Die Mischung wurde in einem Zweischnecken-Extruder (ZSK-40, Fa. Werner + Pfleiderer) unter folgenden Bedingungen extrudiert:
- Temperaturen:
   Schuß 1: 80°C
   Schuß 2: 100°C
   Schuß 3: 130°C
   Schuß 4: 130°C
   Düsen: 135°C
- Materialdurchsatz: 25 kg/h
- Schneckendrehzahl: 160 U/min

Die Schmelze wurde einem Formkalander mit zwei gegenläufig rotierenden Formwalzen (ca. 14 cm nutzbare Formwalzenbreite) zugeführt. Die Vertiefungen der Formwalzen waren so beschaffen, daß aus der Schmelze linsenförmige Tabletten gemäß Fig. 3 geformt wurden.

Die im Rahmen der Erfindung verwendbaren Kalander- und Formwalzen können in an sich bekannter Weise gekühlt oder geheizt werden, und die für den jeweiligen Verarbeitungsprozeß optimale Oberflächentemperatur der Formwalze kann auf diese Weise eingestellt werden.

## Patentansprüche

1. Verfahren zur Herstellung von linsenförmigen Tabletten durch Formen einer wirkstoffhaltigen Schmelze in einem Kalander mit gegenläufig rotierenden Formwalzen, die auf ihrer Oberfläche einander gegenüberliegende Vertiefungen zur Aufnahme und Formung der Schmelze aufweisen, wobei man Formwalzen verwendet, die ellipsoidsegmentartige Vertiefungen aufweisen, **dadurch gekennzeichnet**, daß der Winkel zwischen der Tangentialfläche der Vertiefung am oberen Rand und der Oberfläche der Formwalzen < 45° ist.

2. Verfahren nach Anspruch 1, dadurch gekennzeichnet, daß man Formwalzen verwendet, die kugelsegmentartige Vertiefungen aufweisen.

3. Verfahren nach einem der vorhergehenden Ansprüche, dadurch gekennzeichnet, daß man Formwalzen verwendet, die mit einem Trennmittel versehen sind.

4. Verfahren nach einem der vorhergehenden Ansprüche, dadurch gekennzeichnet, daß man eine wirkstoffhaltige Schmelze verwendet, die mindestens ein pharmazeutisch akzeptables, wasserlösliches Polymer umfaßt.

5. Verfahren nach Anspruch 4, dadurch gekennzeichnet, daß man als Polymer Polyvinylpyrrolidon, ein Copolymerisat von N-Vinylpyrrolidon und einem Vinylester, Copolymerisate von Vinylacetat und Crotonsäure, teilverseiftes Polyvinylacetat, Polyvinylalkohol, ein Ethylen/Vinylacetat-Copolymerisat, Polyhydroxyethylmethacrylat, Copolymerisate von Methylmethacrylat und Acrylsäure, Celluloseester, Celluloseether oder Polyethylenglykol verwendet.

6. Verfahren nach einem der vorhergehenden Ansprüche, dadurch gekennzeichnet, daß man zumindest eine Formwalze verwendet, bei der die Vertiefungen durch mindestens einen Steg unterteilt sind, der sich im wesentlichen bis zur Mantelfläche der Formwalze erstreckt und die Ausbildung einer Bruchrille bewirkt.

7. Kalanderformwalze zur Durchführung des Verfahrens nach einem der Ansprüche 1 bis 6, mit ellipsoidsegmentartiger, Vertiefungen, dadurch gekennzeichnet, daß der Winkel zwischen der Tangentialfläche der Vertiefung am oberen Rand und der Walzenoberfläche < 45° ist.

8. Kalanderformwalze nach Anspruch 7, dadurch gekennzeichnet, daß die Vertiefungen durch mindestens einen Steg unterteilt sind, der sich bis zur Mantelfläche der Formwalze erstreckt.

## Claims

1. A process for the production of lenticular tablets by molding a melt which contains an active ingredient in a calender with counter-rotating molding rolls which have on their surface mutually opposite depressions for receiving and molding the melt, using molding rolls which have depressions in the shape of segments of an ellipsoid, wherein the angle between the tangential surface of the depression at the upper edge and the surface of the molding rolls is < 45°,

2. A process as claimed in claim 1, wherein molding rolls which have depressions in the shape of segments of a sphere are used.

3. A process as claimed in either of the preceding claims, wherein molding rolls provided with a release agent are used.

4. A process as claimed in any of the preceding claims, wherein a melt which contains an active ingredient and which comprises at least one pharmaceutically acceptable, water-soluble polymer is used.

5. A process as claimed in claim 4, wherein polyvinylpyrrolidone, a copolymer of N-vinylpyrrolidone and a vinyl ester, copolymers of vinyl acetate and crotonic acid, partially hydrolyzed polyvinyl acetate, polyvinyl alcohol, an ethylene/vinyl acetate copolymer, poly-(hydroxyethyl methacrylate), copolymers of methyl methacrylate and acrylic acid, cellulose esters, cellulose ethers or polyethylene glycol is used as polymer.

6. A process as claimed in any of the preceding claims, wherein at least one molding roll in which the depressions are divided by at least one bar which extends essentially up to the surface of the molding roll and forms a score is used.

7. A calender molding roll for carrying out the process as claimed in any of claims 1 to 6, with depressions which are in the shape of segments of an ellipsoid, wherein the angle between the tangential surface of the depression at the upper edge and the roller surface is < 45°.

8. A calender molding roll as claimed in claim 7, wherein the depressions are divided by at least one bar which extends up to the surface of the molding roll.

## Revendications

1. Procédé de fabrication de comprimés en forme de lentille par calandrage par formage d'une masse fondue contenant des principes actifs, dans une calandre, avec des rouleaux de formage tournant en sens inverses, présentant sur leurs surfaces des creusements mutuellement opposés destinés à recevoir et à former la masse fondue, en utilisant des rouleaux de formage présentant des cavités du genre de segments d'ellipsoïde, caractérisé par le fait que l'angle entre la surface tangentielle de la cavité sur le bord supérieur et la surface des rouleaux de formage est < 45°.

2. Procédé selon la revendication 1, caractérisé par le fait qu'on utilise des rouleaux de formage qui présentent des cavités du genre de segments sphériques.

3. Procédé selon l'une des revendications précédentes, caractérisé par le fait qu'on utilise des rouleaux de formage qui sont dotés d'un agent séparateur.

4. Procédé selon l'une des revendications précédentes, caractérisé par le fait que l'on utilise une masse fondue contenant des principes actifs, qui comprend au moins un polymère pharmaceutiquement acceptable, soluble dans l'eau.

5. Procédé selon la revendication 4, caractérisé par le fait qu'on utilise comme polymère du polyvinylpyrrolidon, un copolymérisat de N-vinylpyrrolidon et un vinylester, des copolymérisats d'acétate de vinyle et d'acide crotonique, de l'acétate de polyvinyle partiellement saponifié, un alcool de polyvinyle, un copolymérisat éthylène/acétate de vinyle, un polyhydroxyéthylméthacrylate, des copolymérisats de méthylméthacrylate et d'acide acrylique, un ester de cellulose, un éther de cellulose et un polyéthylèneglycol.

6. Procédé selon l'une des revendications précédentes caractérisé par le fait qu'on utilise au moins un rouleau de formage pour lequel les cavités sont subdivisées à l'aide d'au moins une nervure, qui s'étend sensiblement jusqu'à la surface d'enveloppe du rouleau de formage et provoque la formation d'une moulure destinée à favoriser la rupture.

7. Rouleau de formage par calandrage pour la mise en oeuvre du procédé selon l'une des revendications 1 à 6, avec des cavités du genre de segments d'ellipsoïde, caractérisé par le fait que l'angle entre la surface tangentielle de la cavité sur le bord supérieur et la: surface du rouleau est < 45°.

8. Rouleau de formage par calandrage selon la revendication 7, caractérisé par le fait que les cavités sont subdivisées à l'aide d'au moins une nervure qui s'étend jusqu'à la surface d'enveloppe du rouleau de formage.
